# EUROPEAN PATENT APPLICATION

(11) **EP 4 260 881 A1**
(43) Date of publication of application: **18.10.2023**
(21) Application number: 22382358.4
(22) Date of filing: 14.04.2022
(51) Int. Cl.: A61L 9/20

(54) **ULTRAVIOLET RADIATION ABSORBER BY GRANULAR GROUP**

(71) Applicant: Garcia-Gallardo Sanz, Prospero, 09004 Burgos (ES)
(72) Inventor: Garcia-Gallardo Sanz, Prospero, 09004 Burgos (ES)

(57) **Abstract**

The device separates a compartment in which ultraviolet radiation is applied from another compartment in which ultraviolet radiation is not intended to be applied, so that air can circulate between the two compartments by passing through the granular group.

The elements of the group can be of any shape and size; they are made of a material that significantly impedes the rectilinear propagation of the radiation. The gaps between the elements of one layer coincide mostly with the elements of the other layer, and so on, so that a three-dimensional network of air ducts is created, which by changes of direction obstructs the rectilinear path of the radiation and absorbs it by accumulated reflections.

## Description

Emitters of ultraviolet electromagnetic radiation have the potential to inactivate microorganisms and activate certain chemical reactions; for this, a target radiation dose is applied, which is achieved by adjusting the exposure time according to the power of the emitter. Such doses of radiation can be harmful to people, animals, plants, materials, and processes; hence, it is required to locate the radiation emitters in separate compartments from them.

If the aim is to move air between radiation-exposed and non-exposed compartments, then it must be prevented that radiation escapes through the communication openings. The rectilinear path of the radiation must be obstructed and the radiation must be absorbed to the necessary degree by accumulated reflections. So, a path that allows changes in airflow direction is created by positioning perforated and bent surfaces.

The number of required changes of direction is directly related to the power of the radiation emitter to be damped and its distance to the aperture. On the other hand, each change in direction or passage through a perforation implies turbulence that involves friction or loss of pressure in the airflow. Therefore, it is convenient to look for favourable shapes that minimize this effect, but require more space. In any case, these turbulences imply an increase in acoustic emissions and energy consumption.

The proposed invention makes it possible to separate the irradiated from the non-irradiated compartment while allowing the air to circulate between the two compartments through a granular group.

The manufacturing process, space requirements, air friction, acoustic emissions, and energy consumption are different, and often more favourable than in the above-mentioned systems. The granular elements are distributed to fit the shape of any available space defined by the enclosure and the confinement system. The gaps between the elements form multiple ducts that are crossed by the airflow in a reduced turbulence regime, with all its effects. Evidently, there are specific shapes and geometric distributions that are more favourable than others.

### Explanation of the invention:

The device makes it possible to separate a compartment (1) inside which ultraviolet radiation is applied using an emitter (2), from a non-radiated compartment (6), while allowing air flow between the two. Both compartments are separated by an enclosure (5) which prevents the passage of air and ultraviolet radiation and has one or more openings for the passage of air between compartments:
It is characterized by a granular group (3) located in the opening that allows air flow between compartments, and by a confinement system (4) that stabilizes the position of the elements of the group. Figure-1, Figure-2, and Figure-3.

The elements forming the granular group can be of any shape and size. They are made of a material that significantly impedes the rectilinear propagation of radiation. The elements are distributed over the entire surface that is crossed by air, but not by radiation, in a minimum of two layers, with no maximum number of layers and with an ordered or unordered relative arrangement between them; adapting to the shape of the available space defined by the enclosure and the confinement system.

The points of contact between the elements of one layer leave gaps that coincide mostly with elements in the adjacent layer, and so on; this allows the air to flow with changes of direction and obstructs the straight propagation of radiation, which is absorbed by the accumulated reflections. This makes it possible to precisely modify the capacity of radiation absorption by simply modifying the number of elements and layers.

The confinement system allows the passage of air, but not of elements; it can be either external; if it acts as a surface that envelops the group of elements; or internal, if it acts through a mechanism of cohesion between elements. This system can contribute to the geometrical order of the elements of the granular group.

Since the granular elements can be of any shape and size, and have a relative arrangement between them, ordered or disordered, the following variants are included, without the prejudgement that some provide better performance than others:
Considering the shape of the elements:
Convex enclosure elements, ellipsoids or spheres, offer less resistance to the passage of air, and tend to be located on the lower cavities. Thus, the points of contact between the elements of one layer generate spaces that, due to gravity, are occupied by the elements of the adjacent layer. They have shapes comparable to the mathematical quadratic equation 1=((x^2)/a^2))+((y^2)/b^2))+((z^2)/c^2)), for any value of (a), (b) or (c), even if (a=b=c) which is a sphere; and different sizes. Figure-4.

Considering the relative size between elements:
A uniform granulometry of the elements produces a higher ratio of gaps, resulting in a larger section of the air duct network, allowing a higher airflow rate. Figure-5.

Considering the geometric distribution of the elements:
Elements with a convex enclosure and a uniform granulometry can be easily arranged in a geometrical order in each layer so that the ratio of gaps/cavities is homogeneous and the resistance to the passage of air is reduced. Figure-6. The optimal shapes are those of triangular Figure-7 or rectangular designs, Figure-9.

Considering the manufacturing:
The granular elements are distributed to fit the shape of any space defined by the enclosure and the confinement system. Additionally, the confinement system can contribute to the geometrical organization of the elements, by arranging cavities similar to the between-layer gaps generated by the elements distributed in the intended geometry. The optimal shapes are triangular grids Figure-8 or rectangular grids, Figure-10.

Finally, it should be noted that it is possible to distribute the elements using an external confinement system and fix them using an internal binder or mechanism so that the external confinement system can be removed.

### Description of an example of the invention

Take the current situation of the pandemic declared by the World Health Organization (WHO) in March 2020. The SARS-COV-2 virus responsible for causing Covid-19 can be transmitted by aerosols in closed spaces with air recirculation.

The aim is to apply a dose of ultraviolet radiation UVC 254nm of 5 mJ/cm2 on the aerosols that enter a room through an air conduct, with the purpose to destroy the germs without exposing the persons that might be inside the room to the radiation. The compartment to which the radiation is applied is the duct (1), and the compartment to which the radiation is not intended to be applied (6) is the human-occupied room. The enclosure (5) separating the compartments coincides with the enclosure of the duct.

Since the velocity of the air inside the compartment where the radiation is applied is 2m/s, the emitter (2) is required to provide radiation of 10,000 µW/cm2. However, the presence of people for more than 8 hours in the other compartment limits the radiation emission to 0,1µW/cm2. If a material that absorbs 90% and reflects 10% is used, 5 reflections are required, that is, a minimum of 6 layers of elements.

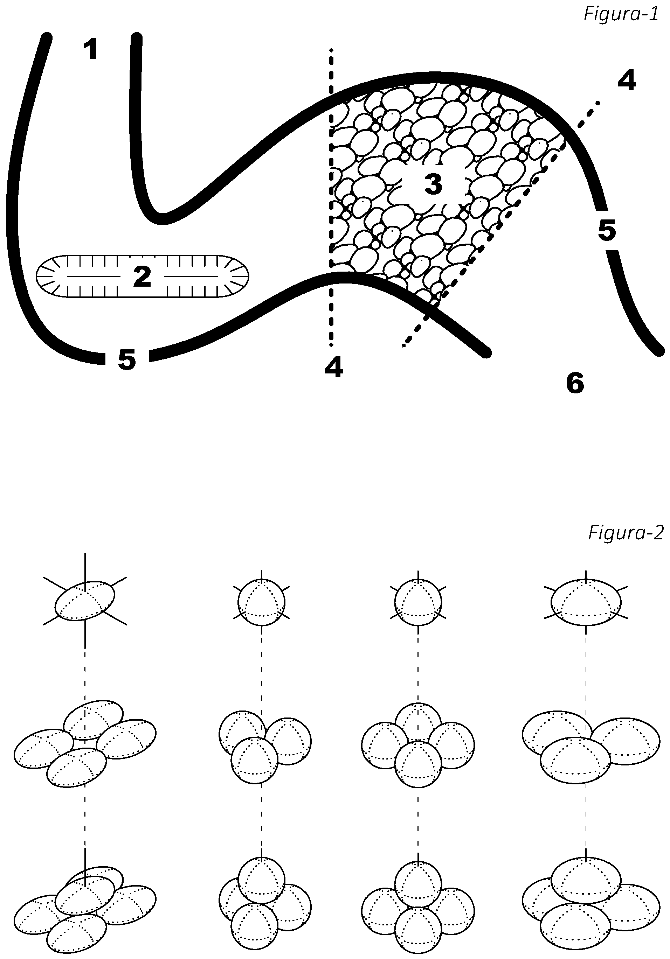

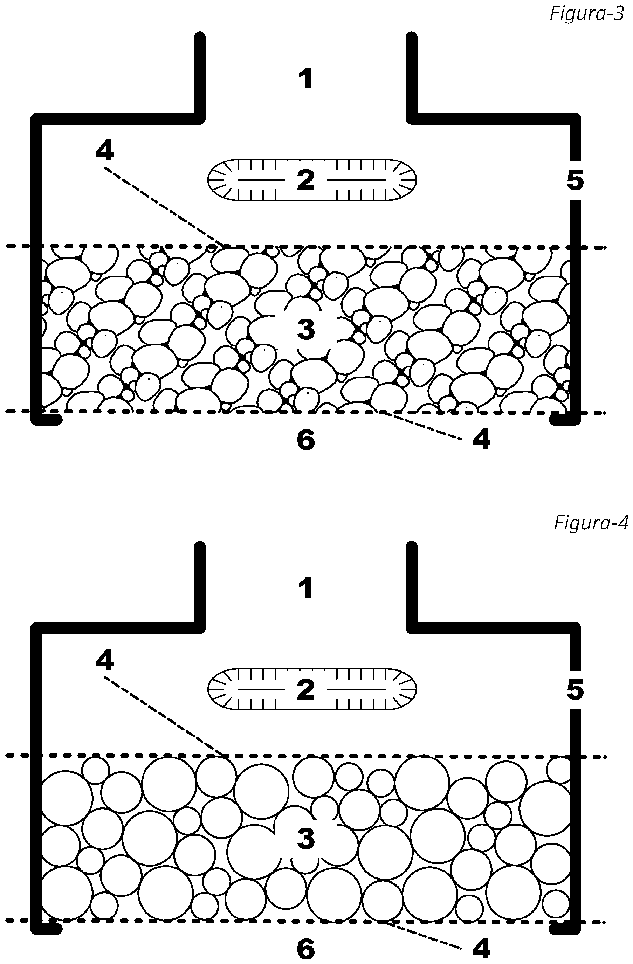

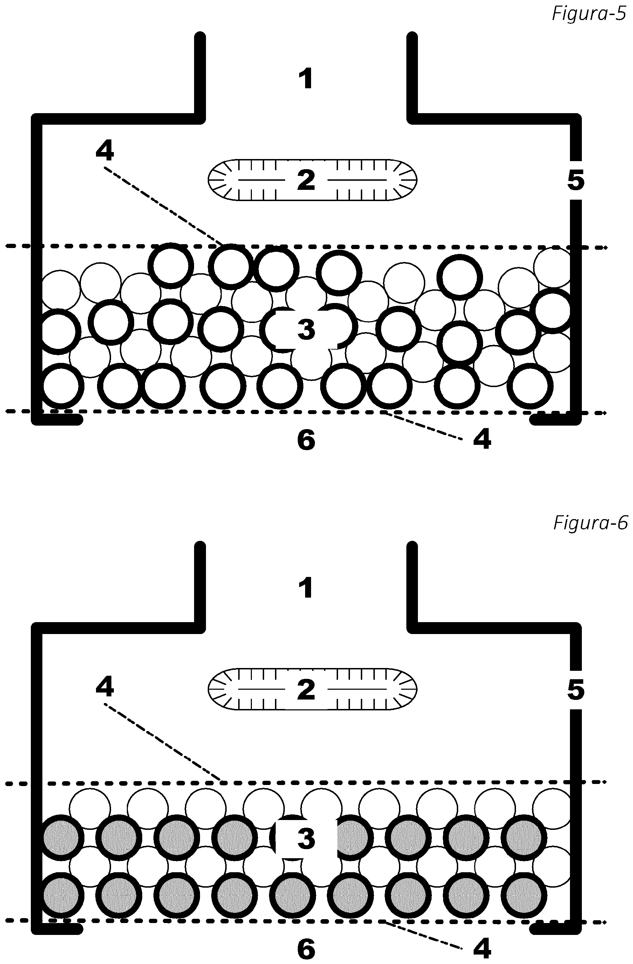

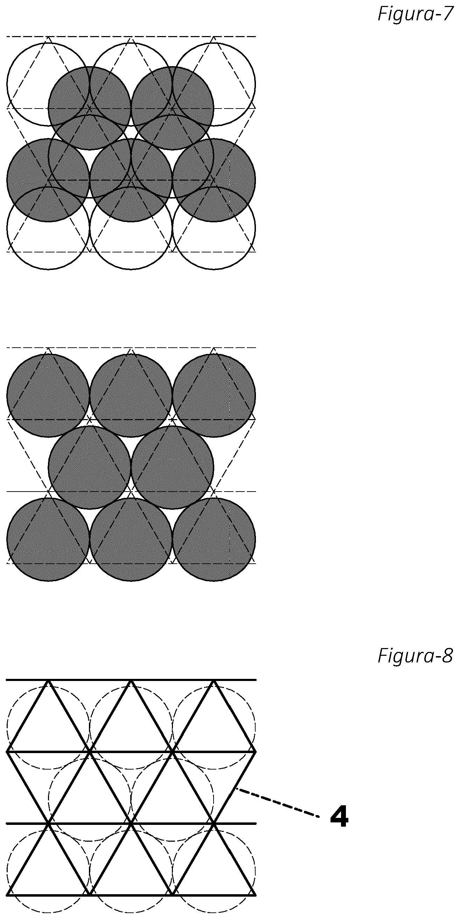

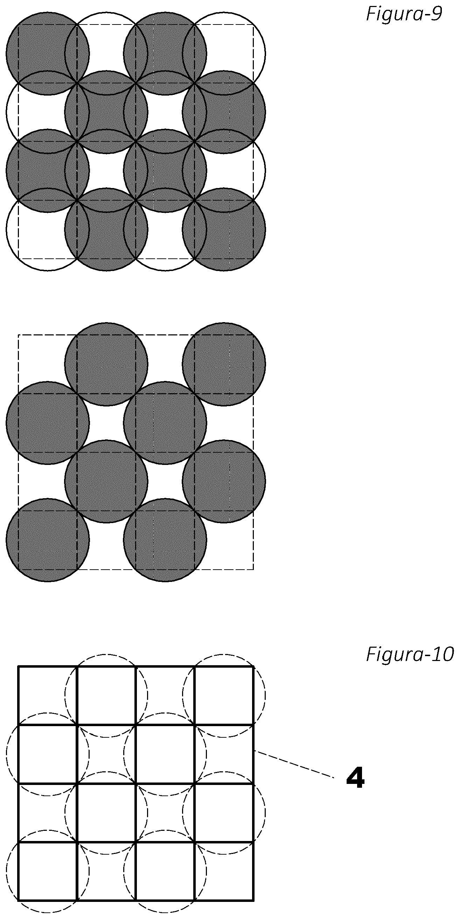

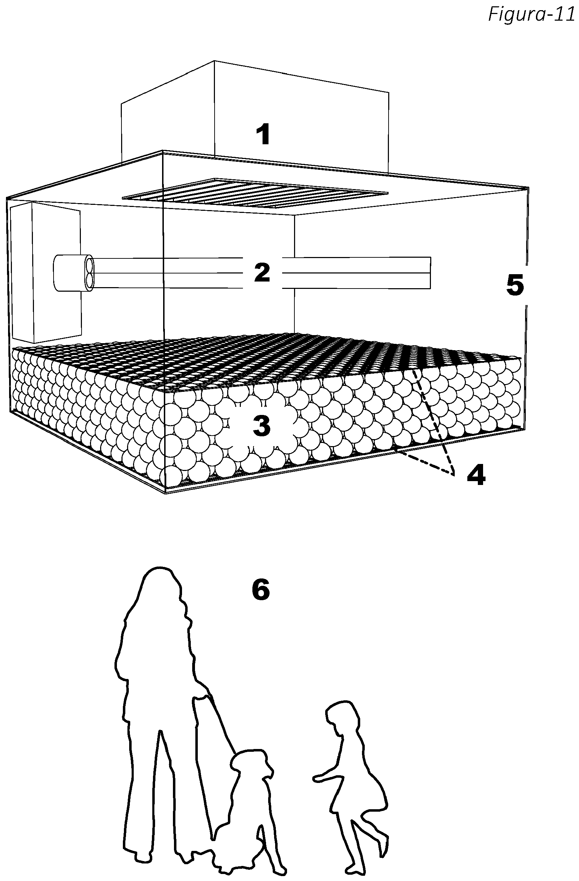

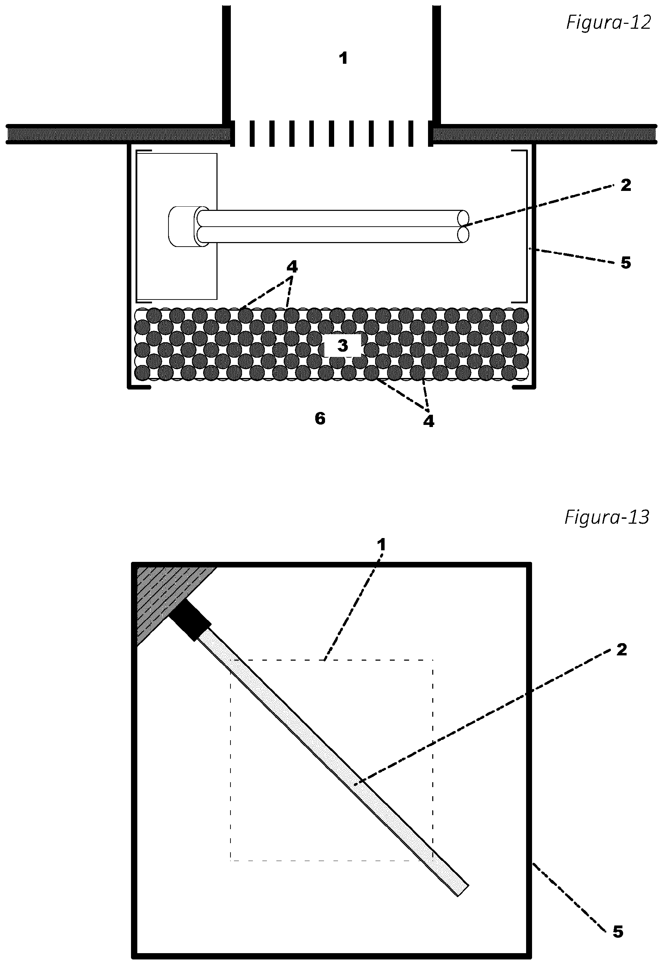

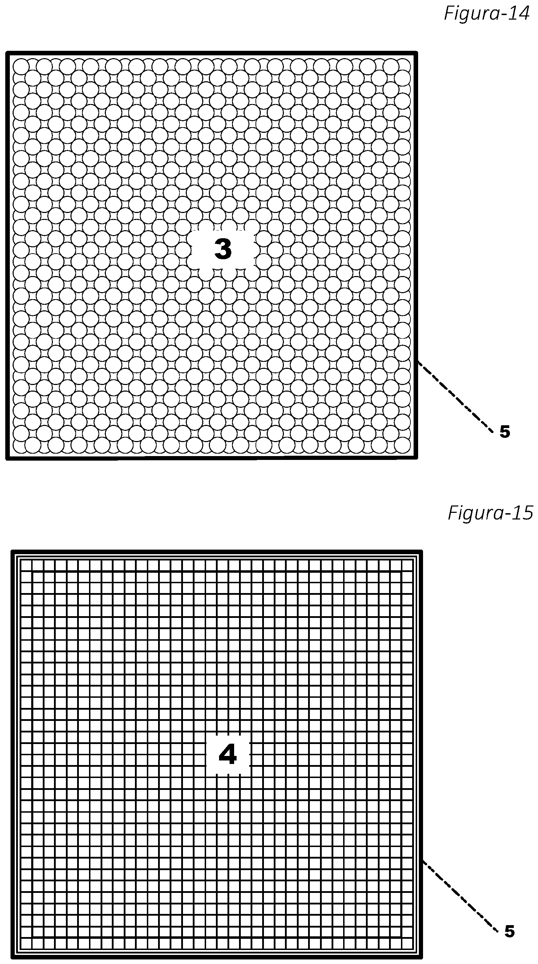

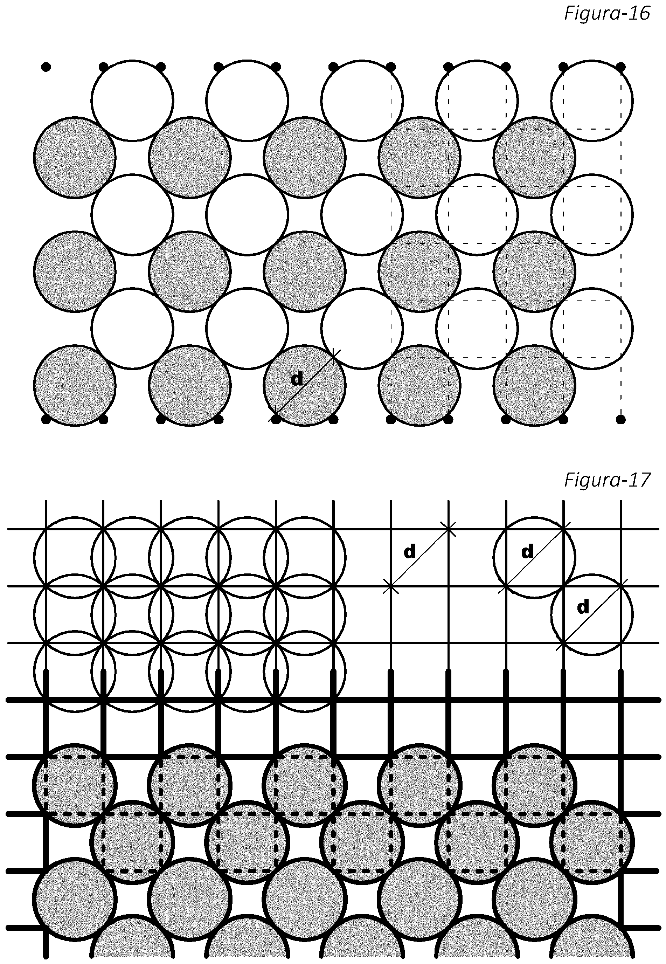

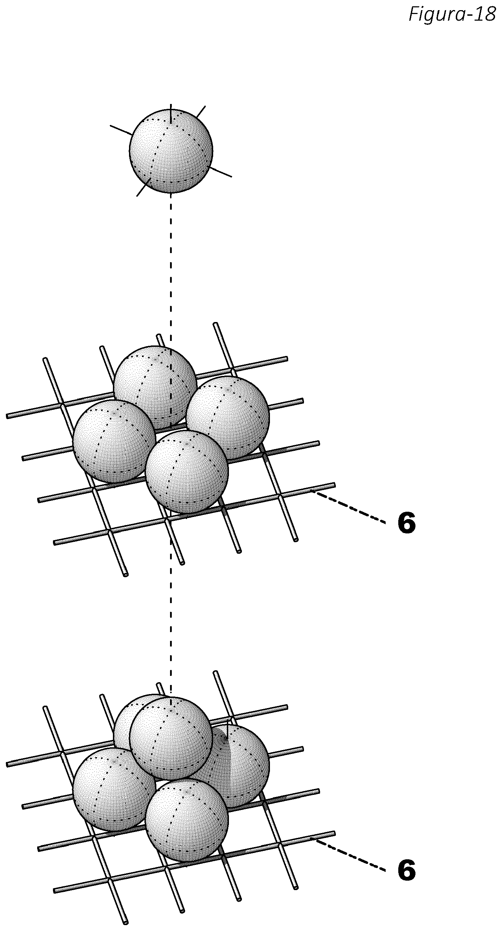

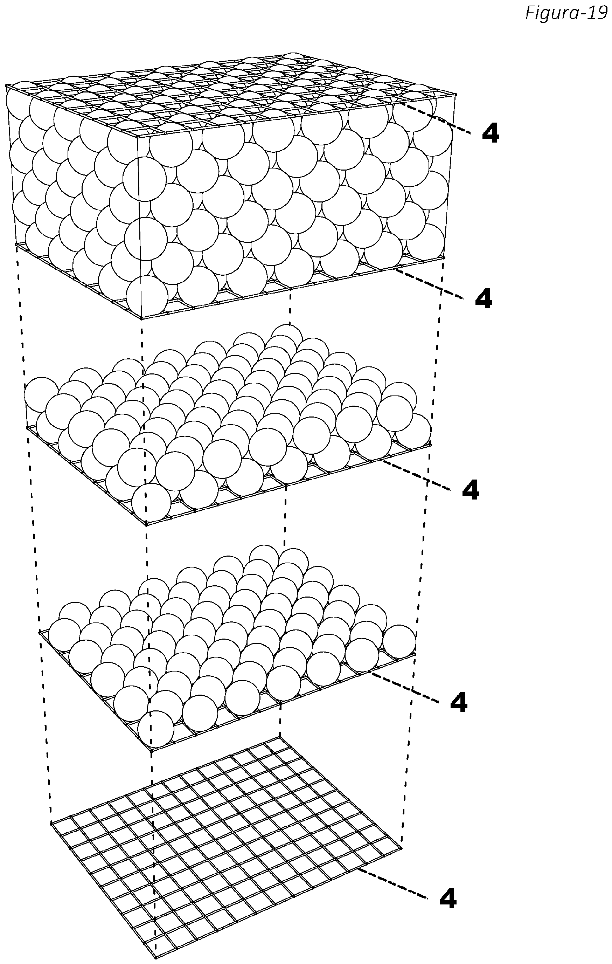

Finally, optimal performance is achieved with the use of a granular group (3) of equal, spherical elements, with a confinement system (4) that contributes to the geometrical order through the arrangement of gaps in a grid of squares, whose diagonal coincides with the diameter of the spheres. This provides a gap ratio varying from 21% to 35% with changes of direction that are spherical and favourable to air circulation. Figures 11 to 19.

### BRIEF EXPLANATION OF THE DRAWINGS

- Figure-1: Conceptual scheme.
- Figure-2: Graphical explanation of how the granular elements of one layer obstruct the rectilinear path of radiation left by the gaps in the other.
- Figure-3: Granular group with elements of any shape and size.
- Figure-4: Granular group with elements of a shape similar to an ellipsoid and different size.
- Figure-5: Granular group with elements of a shape similar to an ellipsoid and the same size.
- Figure-6: Granular group with elements of a shape similar to an ellipsoid, same size and geometrically ordered.
- Figure-7: Granular group with elements similar in shape to an ellipsoid, same size and geometrically arranged in triangular order.
- Figure-8: Granular group with elements similar in shape to an ellipsoid, same size and geometrically arranged in triangular order by means of an external confinement system.
- Figure-9: Granular group with elements similar in shape to an ellipsoid, same size and geometrically arranged in rectangular order.
- Figure-10: Granular group with elements similar in shape to an ellipsoid, same size and geometrically arranged in rectangular order by means of an external confinement system.
- Figure-11: Practical realisation: Sectioned view.
- Figure-12: Practical realisation: Section.
- Figure-13: Practical realisation: Plan by emitter.
- Figure-14: Practical realisation: Plan by the granular group.
- Figure-15: Practical realisation: Plan by the geometric confinement system.
- Figure-16: Practical realisation: Geometrical relationship between the cavities of the confinement system and the elements of the granular group.
- Figure-18: Practical realisation: Graphical explanation of how the granular elements of a layer obstruct the direct propagation of radiation through the voids left by the elements of the adjacent layer.
- Figure-19: Practical realisation: Graphical explanation of how granular elements are arranged in successive layers starting from layers starting from the cavities of the confinement system.

### DESIGNATIONS USED IN THE DRAWINGS

- (1): COMPARTMENT IN WHICH ULTRAVIOLET RADIATION IS APPLIED
- (2): UV MAJORITY WAVE RADIATION EMITTING SYSTEM
- (3): GRANULAR GROUP
- (4): GRANULAR GROUP CONFINEMENT SYSTEM
- (5): ENCLOSURE SEPARATING THE COMPARTMENTS
- (6): COMPARTMENT WHERE ULTRAVIOLET RADIATION IS NOT INTENDED TO BE APPLIED

## Claims

The device makes it possible to separate a compartment (1) inside which ultraviolet radiation is applied by means of an emitter (2) from another compartment (6) to which such radiation is not intended to be applied, so that air can circulate between the two compartments. The two compartments are separated by an enclosure (5) which prevents the passage of air and ultraviolet radiation and has one or more openings for the passage of air between compartments:
It is **characterised by** a granular group (3) located in the opening for the passage of air between compartments, and by a confinement system (4) that maintains the position of the elements of the group stable. Figure-1, Figure-2 and Figure-3.
The elements forming the granular group can be of any shape and size; they are made of a material that significantly impedes the rectilinear propagation of radiation. They are distributed over the entire surface to be traversed by air, but not by radiation; they are distributed in at least two layers, with no maximum number of layers; with relative arrangement between them ordered or disordered; adapting to the shape of any available space defined by the enclosure and the confinement system.
The contact points between the elements of one layer leave gaps that coincide mostly with elements in the adjacent layer, and so on; thus allowing air displacement with changes of direction and obstructing the straight propagation of radiation, which is absorbed by the accumulated reflections. This makes it possible to precisely modify the radiation absorption capacity by simply changing the number of elements and layers.
The confinement system allows the passage of air, but not of elements; it can be external, if it acts as a surface enclosing the group of elements; or internal, if it acts by a cohesion mechanism between elements; and can contribute to the geometrical order of the elements of the granular group.
According to claim one, where the elements can have shapes assimilable to the mathematical equation of the quadric 1=((x^2)/a^2))+((y^2)/b^2))+((z^2)/c^2)); for any value of (a), (b) or (c), including whether (a=b=c) which is a sphere; and different sizes. Figure-4.
According to claim one, where the elements are all of the same size. Figure-5.
According to claim one and three, where the elements are arranged in a geometrically ordered manner, with the gaps between three elements in one layer exactly matching an element in the adjacent layer(s). Figure-6 and Figure-7.
According to claim one and three, where the elements are arranged in a geometrically ordered manner, exactly matching the gaps between four elements of one layer with an element in the adjacent layer(s). Figure-6 and Figure-9.
According to claims four and five, the confinement system contributes to the geometrical organisation of the elements by arranging cavities similar to the gaps generated between layers by the elements distributed in the intended geometry. The optimal shapes are triangular grid Figure-8 or rectangular grid, Figure-10.
According to claim one, the elements can be distributed using an external confinement system, and fixed by a cohesive mechanism between elements; such as an adhesive substance; so that the external confinement system can be removed.
